# EUROPÄISCHE PATENTSCHRIFT

(11) **EP 2 150 184 B1**
(45) Veröffentlichungstag und Bekanntmachung des Hinweises auf die Patenterteilung: **25.09.2019**
(21) Anmeldenummer: 08758663.2
(22) Anmeldetag: 21.05.2008
(51) Int. Cl.: A61B 17/15, A61B 17/17

(54) **VORRICHTUNG ZUR BESTIMMUNG DER MECHANISCHEN BEINACHSE EINES OBERSCHENKELKNOCHENS**
DEVICE FOR DETERMINING THE MECHANICAL LEG AXIS OF A FEMUR
DISPOSITIF DE DÉTERMINATION DE L'AXE MÉCANIQUE DE JAMBE, POUR UN FÉMUR

(30) Priorität: 25.05.2007 DE 102007024708
(43) Veröffentlichungstag der Anmeldung: 10.02.2010
(73) Patentinhaber: MINMAXMEDICAL, 38610 Gières (FR)
(72) Erfinder: RADERMACHER, Klaus, 52222 Stolberg (DE); ELFRING, Robert, 52064 Aachen (DE)
(74) Vertreter: Regimbeau
(86) Internationale Anmeldenummer: PCT/EP2008/004056
(87) Internationale Veröffentlichungsnummer: WO 2008/145287

(56) Entgegenhaltungen:
- WO-A-95/00076
- US-A- 5 690 638

## Beschreibung

Die Erfindung betrifft eine Vorrichtung zur Bestimmung der mechanischen Beinachse eines Oberschenkelknochens, mit einem Schaft, der an seinem einen Ende eine arretierbar winkelvariabel mit dem Schaft verbundene Befestigungsvorrichtung aufweist zur winkelstabil arretierbaren Befestigung des Schaftes am Kniegelenk eines Oberschenkelknochens und an seinem anderen Ende ein Griffelement aufweist, wobei zwischen Griffelement und Schaft eine Kraft-Momenten-Messvorrichtung angeordnet ist, mit der Kraftkomponenten einer mittels des Griffelements über den Schaft auf ein Kniegelenk ausgeübten Kraft messbar sind.

Eine Vorrichtung dieser Art ist z.B. bekannt aus der Veröffentlichung US-A-5 690 638.

Im Bereich der Knieendoprothetik ist es als Problem bekannt, dass Knieprothesen korrekt an der mechanischen Traglinie des Beines, d.h. an der mechanischen Beinachse, der sogenannten Mikuliczlinie ausgerichtet werden müssen. Diese mechanische Beinachse verläuft durch das Sprunggelenkszentrum, das Kniegelenkszentrum und das Hüftkopfzentrum.

Hierbei besteht während einer Operation die Problematik, dass das Hüftkopfzentrum nicht ohne Weiteres ermittelbar ist. Für die korrekte Ausrichtung der femoralen Komponente einer Knieprothese ist dies jedoch von einer zentralen Bedeutung. Nur bei einer gradlinigen Ausrichtung ergibt sich eine instabile Gleichgewichtslage, die durch Bänder und Muskelstrukturen stabilisiert werden können. Abweichungen von dieser idealen gradlinigen Ausrichtung können jedoch zu überhöhten Belastungen, insbesondere in der Kniegelenkprothetik führen, so dass sich hierdurch signifikante Reduzierungen der Lebensdauern künstlicher Kniegelenke ergeben.

Im Stand der Technik ist es beispielsweise als Standard bekannt, zur annähernd richtigen Ausrichtung der femoralen Komponente einer Knieprothetik einen Marknagel in den femoralen Markkanal des Oberschenkelbeinknochens einzuführen. Eine Prothese kann sodann in einem zuvor am Röntgenbild ermittelten Winkel an diesem Nagel ausgerichtet werden, wobei dieser Winkel vorgesehen ist, um die Abweichung der tatsächlichen mechanischen Beinachse von der Richtung des Markkanals zu berücksichtigen. Ein solches Verfahren ist jedoch äußerst invasiv und birgt eine hohe Emboliegefahr aufgrund der Knochenmarkskompression beim Eindringen des Nagels.

Ebenso ist es bekannt, Knieoperationen mit Hilfe von Navigationssystemen durchzuführen, die zu besseren Ergebnissen führen, jedoch einen sehr hohen Aufwand zwischen Einsatz von Planungssoftware und die Notwendigkeit von Positionierungs- oder Trackingsystemen erfordern.

Aufgabe der Erfindung ist es, eine Vorrichtung bereitzustellen, mit denen auf einfache Art und Weise die mechanische Beinachse eines Oberschenkelknochens hinreichend genau bestimmt werden kann, um anhand der so gefundenen und festgelegten mechanischen Beinachse in einer danach stattfindenden Operation eine Knieprothese anpassen zu können. Hierbei ist es weiterhin Aufgabe der Erfindung, eine Vorrichtung bereitzustellen, welche als Vorbereitung bei einer Knieoperation eingesetzt bzw. angewandt werden kann.

Erfindungsgemäß wird diese Aufgabe gelöst durch eine Vorrichtung zur Bestimmung der mechanischen Beinachse eines Oberschenkelknochens mit den im Anspruch 1 angegebenen Merkmalen, weitere besondere Ausführungsformen der Erfindung werden in den Unteransprüchen angegeben. Erfindungsgemäß weist die Vorrichtung einen Schaft auf, der an seinem einen Ende eine arretierbar winkelvariabel mit dem Schaft verbundene Befestigungsvorrichtung aufweist zur winkelstabil arretierbaren Befestigung des Schaftes am Kniegelenk eines Oberschenkelknochens und an seinem anderen Ende ein Griffelement, wobei zwischen Griffelement und Schaft eine Kraft-Momenten-Messvorrichtung angeordnet ist, mit der Kraftkomponenten einer mittels des Griffelements über den Schaft auf ein Kniegelenk ausgeübten Kraft messbar sind, wobei weiterhin mit der Kraft-Momenten-Messvorrichtung Längs- und/oder Querkraftkomponenten der ausgeübten Kraft und/oder dadurch erzeugte Momente messbar sind und die Kraft-Momenten-Messvorrichtung ausgebildet ist als ein erstes ringförmiges Element, welches über wenigstens drei Stege mit einem koaxial darin angeordneten zweiten ringförmigen Element verbunden ist, wobei jeder Steg wenigstens einen Kraftsensor, insbesondere Dehnungsmessstreifen aufweist.

Dabei ist es erfindungsgemäß zumindest vorgesehen, die entstehenden Querkräfte bzw. dadurch erzeugte Momente zu messen, bevorzugt auch die ausgeübte Kraft in axialer Richtung des Schaftes.

Sofern nachfolgend durchführbare Verfahrensschritte benannt werden, stellen diese nicht einen Teil der Erfindung dar, sondern dienen dazu das Verständnis der Erfindung zu erleichtern und ein Beispiel zur Verwendung der erfindungsgemäßen Vorrichtung zu geben.

Es besteht so die Möglichkeit, ein Verfahren zur Bestimmung der mechanischen Beinachse eines Oberschenkelknochens durchzuführen, bei dem ein solcher Schaft, der an seinem Ende arretierbar winkelvariabel mit einer Befestigungsvorrichtung verbunden ist, am distalen Ende eines Oberschenkelknochens, insbesondere am Kniegelenk befestigt wird und sodann über den Schaft eine Kraft auf das distale Ende ausgeübt wird.

Hierbei kann es bevorzugt vorgesehen sein, dass die winkelstabil arretierbare Verbindung zwischen Schaft und Befestigungsvorrichtung als wenigstens ein feststellbares Gelenk, insbesondere ein feststellbares Kugelgelenk ausgebildet ist.

Hierdurch ergibt sich aufgrund der winkelvariable, insbesondere gelenkigen Verbindung zwischen Befestigungsvorrichtung und Schaft ein System mit zwei Gelenken, nämlich gegeben durch das Hüftgelenk und das Gelenk bzw. die winkelvariable Anordnung in der Befestigung, so dass eine Kraft, die auf das distale Ende des Oberschenkelknochens ausgeübt wird, sich in Kraftkomponenten quer zur Richtung der ausgeübten Kraft aufspaltet, sofern die Richtung der ausgeübten Kraft nicht exakt mit der mechanischen Beinachse übereinstimmt.

Solche Querkräfte erzeugen sodann Drehmomente, die mittels der genannten Kraft-Momenten-Messvorrichtung gemessen werden können. Diese Drehmomente wirken dabei im Abstand von der Stelle der Krafteinleitung (Befestigungsvorrichtung am distalen Knochenende) und können daher mit der Kraft-Momenten-Messvorrichtung zwischen Griff und Schaft erfasst werden. Sie wirken daher insbesondere bei der Einspannung der Kraft-Momenten-Messvorrichtung zwischen Griff und Schaft.

Eine Aufspaltung findet nur dann nicht statt bzw. Querkräfte und dadurch erzeugte Momente sind nur dann minimiert oder idealerweise gleich Null, wenn die Kraftlinie der ausgeübten Kraft genau durch beide Gelenke verläuft, d.h. die ausgeübte Kraft exakt in Linie mit der mechanischen Beinachse liegt. So kann sichergestellt werden, dass in dem Augenblick, wenn die gemessenen Querkraftkomponenten minimiert und insbesondere Null sind, der Schaft, mit dem die Kraft auf das distale Ende des Oberschenkelknochens ausgeübt wird, exakt auch in Richtung der mechanischen Beinachse, der sogenannten Mikuliczlinie liegt.

Wenn auch hier in dieser Ausführung von der Feststellung der mechanischen Beinachse gesprochen wird, so kann die Vorrichtung in analoger Weise auch zur Bestimmung anderer Belastungsachsen eingesetzt werden, wie z.B. beim Tibia-Sprunggelenk. Die Erfindung ist nicht auf die Bestimmung der Beinachse beschränkt.

Es ist sodann vorgesehen, dass dann, wenn diese Bedingung erfüllt ist, der Schaft in seiner Position durch Arretierung der winkelvariablen, insbesondere gelenkigen Verbindung zwischen Schaft und Befestigungsvorrichtung fixiert wird. Der fixierte Schaft hat dann eine Längserstreckung, die mit der mechanischen Beinachse übereinstimmt oder zu dieser eine bekannte vordefinierte Lagebeziehung hat, insbesondere so, dass eine entsprechende Vorrichtung die Längsachse reproduzieren kann.

Eine solche Arretierung kann z.B. durch eine Fixierung des wenigstens einen Gelenks, z.B. Kugelgelenks erfolgen. Eine Fixierung kann alternativ auch derart erfolgen, dass eine entsprechende Referenz am Bein fixiert wird, die die Längsachse definiert oder zu dieser eine definierte bekannte Lagebeziehung hat, z.B. durch Einschießen von Kirschnerdrähten, "Bärenkralle", o.ä..

Um dies zu erzielen, ist es erfindungsgemäß vorgesehen, die Kraftkomponenten öder dadurch erzeugte Momente mittels einer Kraft-Momenten-Messvorrichtung aufzunehmen, die direkt am Schaft angeordnet ist und über die eine Kraft, insbesondere zum Schaft axiale Kraft auf den Schaft ausgeübt wird.

Hierbei kann es beispielsweise vorgesehen sein, dass die bei der Ausübung der Kraft entstehenden Querkräfte und/oder dadurch erzeugte Momente mittels der Kraft-Momente-Messvorrichtung erfasst werden. Diese können z.B. in einer Ausführung elektronisch qualitativ und/oder quantitativ erfasst werden und zu einer Anzeige gebracht werden, beispielsweise auf einem Monitor oder einem sonstigen Display. In einer anderen Ausführung können die Kräfte oder Momente auch rein mechanisch gemessen und in einem Display, z.B. Zeigerdisplay) angezeigt werden.

Dies ist besonders komfortabel, da unmittelbar an dem Display sich ändernde Kraft- oder Momentenverhältnisse, wie z.B. Größen und Richtungen der Querkraftkomponenten oder Momente ersichtlich sind und die Krafteinleitung über den Schaft aufgrund der winkelvariablen, insbesondere gelenkigen Verbindung mit der Befestigungsvorrichtung in der Richtung geändert werden kann, bis dass die Bedingung der verschwindenden Querkraftkomponenten / Momente erzielt ist.

Hierfür weist eine erfindungsgemäße Vorrichtung zur Durchführung eines solchen Verfahrens ein Griffelement auf, welches geeignet ist, um die Kräfte über den Schaft auf den Oberschenkelknochen manuell auszuüben. Anhand des Griffelementes kann die Ausrichtung der gesamten Vorrichtung relativ zum distalen Ende des Oberschenkelknochens und hier insbesondere zu der Befestigungsvorrichtung aufgrund der winkelvariablen, insbesondere gelenkigen Verbindung mit dieser geändert werden.

Bei der erfindungsgemäßen Vorrichtung kann es weiterhin vorgesehen sein, dass der Betrag der ausgeübten Kraft auf einer Anzeige visualisiert wird, insbesondere in Relation zu einer Mindestkraft. Dies wird bevorzugt, da nur bei Ausübung einer gewissen Mindestkraft genügend große Querkraftkomponenten und Momente bei einer Fehlausrichtung entstehen, die geeignet sind, die Fehlausrichtung zu visualisieren und zu korrigieren. Beispielsweise kann die Anzeige einer ausreichenden Kraft visualisiert werden durch einen Farbumschlag in einer Anzeige, z.B. von rot nach grün. Hierbei visualisiert der grüne Farbumschlag, dass die notwendige Mindestkraft erreicht ist. Je größer diese Kraft, insbesondere innerhalb sinnvoller Grenzen gewählt wird, desto größer ist die Genauigkeit der Ausrichtung aufgrund entsprechend ausgeprägter Querkräfte und/oder Momente.

Die Querkraftkomponenten bzw. die hierdurch erzeugten Momente können in einer Anzeige beispielsweise als ein Fadenkreuz bzw. dessen relative Lage zu einem Zentrum in der Anzeige visualisiert werden. Dies ist beispielsweise dann vorteilhaft, wenn ein kartesisches Koordinatensystem zugrunde gelegt wird und beispielsweise die Richtung der ausgeübten Kraft als Z-Richtung angenommen wird, wobei dann Querkraftkomponenten in X- und Y-Richtung durch die Messvorrichtung erfasst werden können. So kann der Betrag der Querkraftkomponenten und/oder Momente durch die Lage eines Fadenkreuzes in einem kartesischen Koordinatensystem relativ zum Ursprung visualisiert werden.

In dem Augenblick, wenn das Fadenkreuz in den Ursprung gelegt ist, ist somit sichergestellt, dass die Querkraftkomponenten bzw. Momente minimiert, idealerweise gleich Null sind und somit der Schaft, über den in dieser Lage die Kraft auf das distale Ende des Oberschenkelknochens ausgeübt wird, die ideale Lage hat, d.h. in Verlängerung der Mikuliczlinie liegt. Hierbei kann es vorgesehen sein, dass der vorgenannte Farbumschlag beispielsweise direkt anhand der Farbgebung des Fadenkreuzes visualisiert wird.

Eine Anzeige kann auch in einer beliebigen anderen Anzeigevorrichtung realisiert sein, z.B. einer die direkt an der Vorrichtung angeordnet ist, z.B. am Griffelement.

Diese Anzeige kann auch rein mechanisch über entsprechende Messvorrichtungen, insbesondere mit mechanischen Zeigerinstrumenten ausgebildet sein. Hier kann ggfs. nur visualisiert werden, in welche Richtung eine Bedienperson den Griff verlagern muss, um zur idealen Position zu gelangen.

Um hierbei Fehlmessungen zu vermeiden, die dadurch resultieren können, dass gegebenenfalls das distale Ende des Oberschenkelknochens eine Kraftkomponente oder ein Moment aufgrund der wirkenden Schwerkraft in der Anzeige hervorruft, kann es vorgesehen sein, dass vor der Durchführung der eigentlichen Messung zur Bestimmung der Beinachse die wirkende Gewichtskraft durch die Kraft-Momenten-Messvorrichtung ermittelt und sodann bei folgenden Messungen berücksichtigt wird. So kann eine Kompensation der wirkenden Gewichtskraftkomponente erfolgen.

Eine erfindungsgemäße Vorrichtung zur Durchführung dieses Verfahrens weist wie eingangs genannt einen Schaft auf, der mit seiner einen Seite über eine winkelvariable, insbesondere gelenkige Verbindung, die durch eine Befestigungsvorrichtung gegeben wird, mit dem distalen Ende eines Oberschenkelknochens verbunden wird. Über ein Griffelement am anderen Ende des Schaftes kann so die eingangs beschriebene Kraft auf das distale Ende ausgeübt werden.

Hierbei ist die Kraft-Momenten-Messvorrichtung zwischen Griffelement und Schaft angeordnet. Beispielsweise kann eine solche Anordnung lösbar gestaltet sein, wenn die Kraft-Momenten-Messvorrichtung beispielsweise zwischen zwei Flanschen angeordnet ist. Hierbei kann einer der beiden Flansche dem Griffelement und der andere dem Schaft zugeordnet sein.

In einer bevorzugten Ausführung der erfindungsgemäßen Vorrichtung kann es vorgesehen sein, dass der Schaft wenigstens zweiteilig und hierbei insbesondere teleskopierbar ausgebildet ist. Hierbei kann ein Teil des Schaftes mit der Befestigungsvorrichtung wie eingangs erwähnt arretierbar winkelvariabel, insbesondere gelenkig verbunden sein, wobei ein anderer Teil mit der Griffvorrichtung bzw. der Kraft-Momenten-Messvorrichtung und beide Teile miteinander lösbar verbindbar sind. Hierbei kann eine eventuelle Teleskopierbarkeit in der lösbaren Verbindung der beiden Teile untereinander realisiert sein. Die Teleskopierbarkeit hat hierbei den besonderen Vorteil, dass die Länge der gesamten Vorrichtung sowie insbesondere die Länge des Schaftes angepasst werden kann an vorherrschende Bedingungen oder an Präferenzen der bedienenden Person.

Die wenigstens zweiteilige Ausführung des Schaftes hat dabei den Vorteil, dass die Verbindung zwischen diesen beiden Teilen gelöst werden kann, so dass nach einer winkelstabilen Arretierung des Schaftes in der Befestigungsvorrichtung der Rest der erfindungsgemäßen Vorrichtung von dem arretierten Schaftstück entfernt werden kann. Das sodann arretierte und an dem distalen Ende des Oberschenkelknochens winkelstabil befestigte Schaftende kann sodann eingesetzt werden, um in einer nachfolgenden Operation eine Knieprothese an das distale Ende des Oberschenkelknochens anzupassen. Dies kann hierbei insbesondere auf bekannte Art und Weise erfolgen.

In einer weiteren bevorzugten Weiterbildung kann es auch vorgesehen sein, dass der Schaft vor der Befestigungsvorrichtung, also insbesondere der vorgenannte Teil, der mit der Befestigungsvorrichtung verbunden ist, geknickt oder gebogen ausgeführt ist, insbesondere so, dass ein vorderer und ein hinterer Teil des Schaftes (vor und hinter der Biegung / Knickung) in einer Linie liegen. Hierbei kann die Ausführung der Biegung oder gegebenenfalls mehrfachen Knickung derart erfolgen, dass der Schaft im Bereich der Biegung/Knickung ein Hindernis, z.B. eine Kniescheibe umgibt bzw. umläuft. So kann eine solche Vorrichtung bevorzugt auch minimal-invasiv eingesetzt werden.

Um die eingangs genannte Arretierbarkeit der winkelvariablen, insbesondere gelenkigen Verbindung zwischen Schaft und Befestigungsvorrichtung zu realisieren, kann es in einer besonders bevorzugten Ausführung vorgesehen sein, dass der Schaft zumindest teilbereichsweise hohl ausgebildet ist, wobei durch den hohlen Schaft hindurch eine Arretiervorrichtung für die Arretierung der winkelvariablen, insbesondere gelenkigen Verbindung zur Befestigungsvorrichtung lösbar und/oder feststellbar ist. Beispielsweise kann dies erfolgen, indem im hohlen Schaft ein insbesondere mehrteiliger Stift einliegt, mittels dem die Arretiervorrichtung in der Befestigungsvorrichtung betätigbar ist.

Hier kann es beispielsweise vorgesehen sein, dass der Stift durch ein Betätigungselement betätigbar ist, welches am Griffelement vorgesehen ist. Dies hat den besonderen Vorteil, dass eine bedienende Person mittels des Griffelementes die Kraft über den Schaft auf das distale Ende des Oberschenkelknochens ausüben kann und in dem Augenblick, wenn die Bedingung der minimierten Querkraftkomponenten eintritt, an dem Betätigungselement die Arretierung bewirken kann. Dabei kann es vorgesehen sein, dass die Fixierung durch Betätigung des Betätigungselements ausgelöst wird, das Betätigungselement also für die Dauer der Ausrichtung des Schaftes unbetätigt bleibt. Alternativ kann als Standardeinstellung eine Fixierung gegeben sein, die durch Betätigung des Betätigungselements für die Dauer der Ausrichtung gelöst wird. Wird das Betätigungselement dann z.B. losgelassen erfolgt automatisch die Fixierung.

Als besonders ergonomisch und vorteilhaft wird eine erfindungsgemäße Vorrichtung dabei empfunden, wenn Griffelement und Schaft, insbesondere auch das Betätigungselement im Griffelement pistolenartig ausgebildet sind. Sodann kann eine bedienende Person die erfindungsgemäße Vorrichtung wie in der Art mit einer Pistole diese in die Hand nehmen und die Messkraft auf das distale Ende des Oberschenkelknochens ausüben. Die Auslösung kann dann durch einen oder mehrere Finger der bedienenden Hand an dem vorgenannten Betätigungselement erfolgen, um den Schaft in seiner Lage zu fixieren.

Um die eingangs genannte arretierbare winkelvariable, insbesondere gelenkige Verbindung zwischen Schaft und Befestigungsvorrichtung zu realisieren, kann es vorgesehen sein, dass diese Verbindung als ein Kugelgelenk ausgebildet ist. Dies hat den besonderen Vorteil, dass hierdurch eine gelenkige Verbindung in zwei Dimensionen, insbesondere innerhalb eines Kegelbereiches um den Kugelmittelpunkt erfolgen kann. So ergeben sich für die bedienende Person vielfältige Möglichkeiten innerhalb des durch die Vorrichtung gegebenen Kegels, der alle möglichen Positionen des Schaftes innerhalb der Befestigungsvorrichtung begrenzt, auszurichten.

Hierbei kann eine Arretierung der kugelgelenkartigen Verbindung beispielsweise derart erfolgen, dass die Kugel gegen ein kugelschalenförmiges Element durch eine Feder vorgespannt ist und diese Federvorspannung beispielsweise während der Ausrichtung der Vorrichtung in die ideale Lage reduziert wird und dann, wenn eine Arretierung gewünscht ist, diese Reduktion aufgehoben wird. Hierdurch kann ein Presssitz zwischen Kugel und Kugelschale erfolgen, in welcher die Kugel gelagert ist. Durch die dadurch erzeugte Reibung kann eine ausreichende Fixierung des Schaftes erzielt werden.

Die hier zum Einsatz kommende Kraft-Momenten-Messvorrichtung kann bevorzugterweise als ein separates Bauelement realisiert sein, welches wie bereits eingangs genannt lösbar zwischen Griffelement und Schaft angeordnet sein kann. Erfindungsgemäß ist es vorgesehen, dass die Kraft-Momenten-Messvorrichtung ausgebildet ist als ein erstes ringförmiges Element, welches über wenigstens drei Stege mit einem koaxial darin angeordneten zweiten ringförmigen Element verbunden ist, wobei jeder Steg wenigstens einen Kraftsensor, insbesondere Dehnungsmessstreifen, aufweist.

So können an den Dehnungsmessstreifen, die an den Stegen vorgesehen sind, auf elektronische Weise Signale abgegriffen werden, die wirkende Kräfte zwischen den beiden ringförmigen Elementen repräsentieren. So können diese Signale ausgewertet werden, um wie eingangs genannt eine Anzeige anzusteuern, um einem Benutzer die Größe der Querkraftkomponenten zu visualisieren.

Hierbei kann es für eine Befestigung vorgesehen sein, dass das erste ringförmige Element mit dem Griffelement und das zweite ringförmige Element mit dem Schaft verbunden ist bzw. bei einem separaten Bauteil zumindest verbindbar ist. Insbesondere die Ausbildung als separates Bauteil hat dabei den Vorteil, dass die Vorrichtung demontierbar und insgesamt sterilisierbar ist, beispielsweise in einem Autoklaven. So kann es erfindungsgemäß vorgesehen sein, dass die Vorrichtung insgesamt oder zumindest deren Komponenten autoklavierbar sind.

Die eingangs genannte Kraft-Momenten-Messvorrichtung weist aufgrund der ringförmigen Ausgestaltung des koaxial innen liegenden zweiten Ringes noch weiterhin den Vorteil auf, dass ein Stift zur Betätigung der Arretiervorrichtung, wie er eingangs genannt ist und innerhalb des hohlen Schaftes verlaufen kann, durch dieses zweite ringförmige Element hindurchdringen kann. So kann insgesamt mit einer derartigen Kraft-Momenten-Messvorrichtung und der erfindungsgemäßen Vorrichtung sichergestellt werden, dass die Richtung der ausgeübten Kraft immer zentral durch die Messvorrichtung verläuft.

Ein Ausführungsbeispiel der Erfindung ist in den nachfolgenden Figuren dargestellt. Es zeigen
- Figur 1: die Kraftsituation beim Einsatz der erfindungsgemäßen der Vorrichtung
- Figur 2 -: eine technische Querschnittszeichnung einer erfindungsgemäßen Vorrichtung
- Figur 3: eine Kraftmesseinrichtung in zwei Ansichten
- Figur 4: eine erste Ausführung eines Schaftes mit Knicken, um eine Kniescheibe zu umgeben
- Figur 5: eine zweite Ausführung eines Schaftes mit Knicken, um eine Kniescheibe zu umgeben

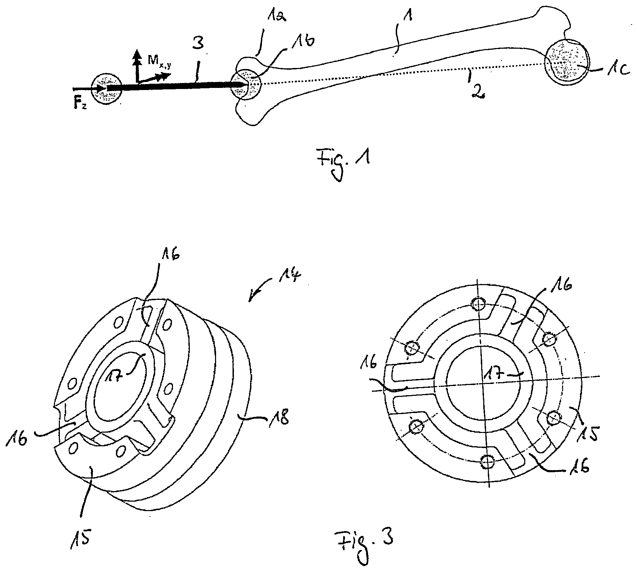

Die Figur 1 zeigt in schematischer Darstellung einen Oberschenkelknochen 1, an dessen distalen Ende 1a eine Knieprothese angebracht werden soll. Erkennbar ist hier, dass die Verbindungslinie 2, die sogenannte Mikuliczlinie oder mechanische Beinachse des Oberschenkelknochens 1, die das Kniegelenk 1b mit dem Hüftgelenk 1c verbindet, außerhalb der Längserstreckung des Knochens 1 liegt.

Die Erfindung hat sich zur Aufgabe gesetzt, diese ideale Linie 2 mit der erfindungsgemäßen Vorrichtung aufzufinden, wofür es vorgesehen ist, eine Kraft FZ in einer hier angenommenen Richtung Z mittels der erfindungsgemäßen Vorrichtung und hier insbesondere über einen Schaft 3 auf das Kniegelenk 1b auszuüben, wofür das Ende des Schaftes 3 mittels einer Befestigungsvorrichtung am Kniegelenk 1b befestigt ist und das Ende des Schaftes 3 innerhalb dieser Befestigungsvorrichtung gelenkig angeordnet ist.

Wird somit die Kraft FZ an einem hinteren Ende des Schaftes 3, z.B. über ein Griffelement auf das Kniegelenk 1b ausgeübt, so wird dann, wenn die Richtung der Kraft FZ nicht exakt mit der Mikuliczlinie 2 übereinstimmt, die wirkende Kraft in zwei Komponenten X und Y aufgespalten. Hierdurch ergeben sich am Ort der Kraft-Momenten-Messvorrichtung messbare Drehmomente. Erfindungsgemäß ist vorgesehen, diese Kraftkomponenten und/oder die hierdurch erzeugten Momente zu erfassen und zur Anzeige zu bringen. Dann, wenn diese Kraftkomponenten / Momente minimiert bzw. ideal Null sind, ist sichergestellt, dass die Richtung der Kraft FZ mit der Mikuliczlinie 2 übereinstimmt und somit die Richtung des Schaftes 3 ideal für eine später folgende Operation ist. Die Lage des Schaftes 3 kann sodann erfindungsgemäß fixiert werden.

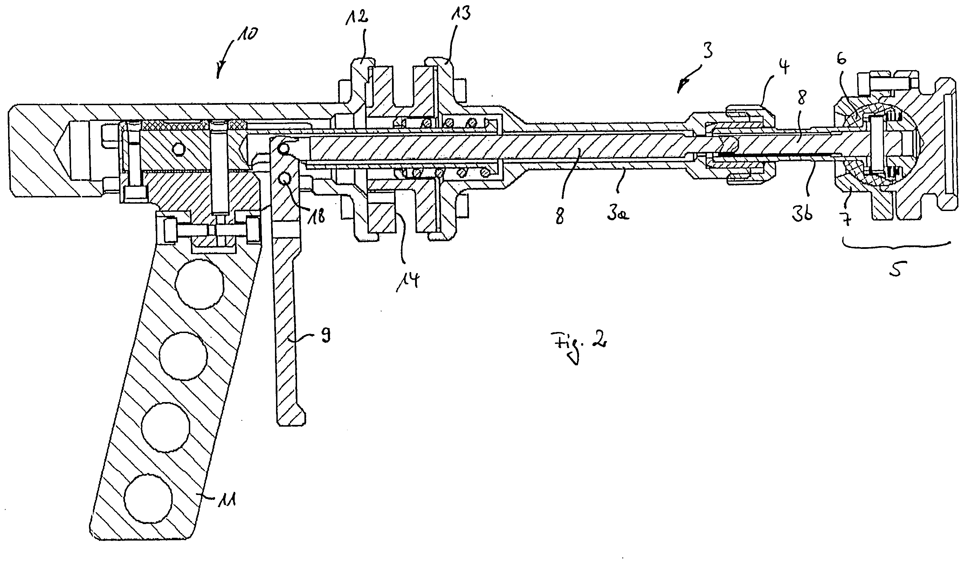

Die Figur 2 zeigt in einer technischen Schnittdarstellung eine erfindungsgemäße Vorrichtung, wie sie zur Durchführung des Verfahrens eingesetzt werden kann. Erkennbar ist in dieser Figur 2 eine etwa pistolenartig ausgestaltete Vorrichtung mit einem Schaft 3, der in zwei Teile 3a und 3b unterteilt ist. Der Teil 3b ist mit dem Teil 3a über eine Verbindung 4, verbunden, wobei diese als Schraubverbindung ausgestaltet sein kann.

Weiterhin ist der Teil 3b gelenkig mit einer Befestigungsvorrichtung 5 verbunden, die dafür vorgesehen ist, zumindest zeitweise am Kniegelenk befestigt zu werden. Bei der hier dargestellten gelenkigen Befestigung ist das hier rechtsseitige Ende des Schaftes 3b mit einem zumindest zum Teil kugelförmigen Element 6 verbunden, welches in einer Kugelschale 7 drehbar gelagert ist, die ihrerseits an der Befestigungsvorrichtung angeordnet ist. Das Kugelelement 6 ist hierbei durch Federn nach links vorgespannt bzw. kraftbelastet, so dass das Kugelelement 6 durch diese Federkraft gegen die Kugelschale 7 gepresst und so in seiner Lage fixiert wird.

Erkennbar ist es hier in der Figur 2, dass der Schaft 3 hohl ausgestaltet ist, wobei der Schaft von einem Stift 8 durchdrungen ist, der an der Verbindungsstelle 4 ebenfalls eine Zweiteilung aufweist. Der Stift 8 ist über einen Betätigungshebel 9, der an einem Griffelement 10 angeordnet ist, in axialer Richtung innerhalb des Schaftes 3 bewegbar, wobei durch das Ziehen des Hebels 9 auf den Griff 11 zu der Stift 8 nach rechts verschoben wird und hierdurch entweder die Federvorspannung auf das Kugelelement 6 reduziert oder in anderer Ausführung das Kugelelement aus der Kugelschale gedrückt wird und somit der Schaft 3 in seiner Bewegung um die Kugelmitte frei wird.

Zwischen dem Griffelement 10 und dem Schaft 3 ist hier zwischen zwei Flanschen 12 und 13 eine Kraft-Momenten-Messvorrichtung 14 angeordnet, die in der Figur 3 näher dargestellt ist. Mittels dieser Kraft-Momenten-Messvorrichtung 14 kann sowohl die wirkende Gesamtkraft, die über das Griffelement 10 und den Schaft 3 auf das distale Ende des Oberschenkelknochens ausgeübt wird, gemessen werden, ebenso wie Querkraftkomponenten, insbesondere solche, die senkrecht zur ausgeübten Kraftrichtung liegen, bzw. die hierdurch am Ort der Kraft-Momenten-Messvorrichtung erzeugten Momente.

Die Figur 3 zeigt in zwei verschiedenen Ansichten eine Kraft-Momenten-Messvorrichtung 14 gemäß der Erfindung. Diese Kraft-Momenten-Messvorrichtung umfasst einen ersten Ring 15, der mit einem der Flansche 12 oder 13 verbindbar ist und der über hier drei Stege 16 mit einem inneren Ring 17 verbunden ist. Dieser innere Ring 17, der koaxial innerhalb des ersten ringförmigen Elementes 15 angeordnet ist, geht dabei in ein weiteres Ringelement 18 über, welches im Wesentlichen denselben Durchmesser aufweist wie das erste ringförmige Element 15. Dieses weitere ringförmige Element 18 kann mit dem anderen Flansch, d.h. einem der beiden Flansche 12 oder 13 verbunden werden, so dass die hier dargestellte Kraftmesseinrichtung zwischen Griffelement 10 und Schaft 3 gemäß der Figur 2 angeordnet werden kann. Die weiterhin in der Figur 2 dargestellte Anordnung eines Stiftes 8 kann hier den inneren freien Bereich des Ringes 17 durchdringen.

Auf den Stegen 16 können bei dieser Ausführung beispielsweise Dehnungsmessstreifen angeordnet sein, wobei es hier in bevorzugter Ausführung vorgesehen sein kann, pro Steg wenigstens zwei Dehnungsmessstreifen (auf verschiedenen Seiten des Stegs) einzusetzen. Anhand dieser Dehnungsmessstreifen können die eingangs genannten Querkraftkomponenten elektronisch erfasst und ausgewertet werden. Beispielsweise können die Messsignale einer Anzeigevorrichtung zugeführt werden. Die visualisierten Messsignale, z.B. in Form eines Fadenkreuzes, könnten sodann einer bedienenden Person eine Hilfestellung bilden, um den Schaft 3 entlang der Mikuliczlinie auszurichten, was dann erfolgt, wenn die Querkraftkomponenten minimiert bzw. Null sind.

Mit Bezug auf die Figur 2 wird der Schaft 3 bzw. zumindest dessen rechtsseitiges Teil 3b in der Befestigungsvorrichtung 5 fixiert, wenn der Griff 9 gelöst wird, d.h. in Bezug auf die Figur 2 sich von dem Handgriff 11 entfernt. Der Griff 9 ist an einer Achse 19 am Griffelement drehbar gelagert, so dass dessen oberes Ende beim Lösen des Betätigungselementes 9 nach links zurückweicht und so der Stift 8 innerhalb des Schaftes 3 ebenso nach links gezogen wird. Hierdurch wird bewirkt, dass die Federvorspannung wieder vollständig auf das Kugelelement 6 innerhalb der Befestigungsvorrichtung wirkt und dieses somit gegen die Kugelschale 7 gedrückt und somit in seiner Lage fixiert wird.

Wenn die Lage des Schaftes 3 ausreichend genau fixiert ist, besteht hier die Möglichkeit, die Verbindung 4 zwischen den beiden Teilen 3a und 3b des Schaftes 3 zu lösen, so dass nur der fixierte Teil 3b am distalen Ende des Oberschenkelknochens verbleibt. Dieser verbleibende Teil 3b des Schaftes kann sodann als Montagestift verwendet werden, um in einer nachfolgenden Operation eine Knieprothese anzupassen.

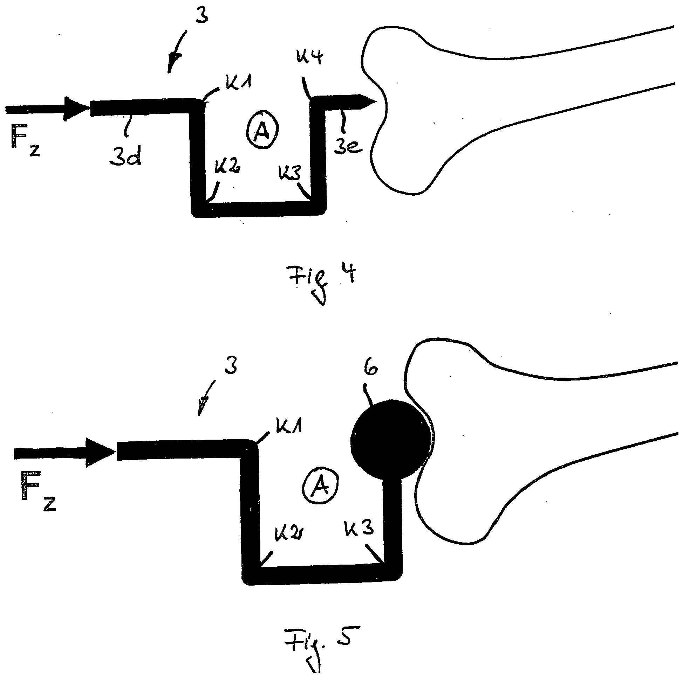

Die Figur 4 zeigt eine Ausführung, bei der ein Schaft in seinem der Befestigungsvorrichtung nahen Teil mehrere Knicke K1, K2, K3 und K4 aufweist. Die vor und hinter den Knicken liegenden Schaftabschnitte 3d und 3e liegen in einer Linie und fluchten. Durch die Knicke wird eine Ausnehmung definiert, in der eine Kniescheibe einliegen kann, so dass der Schaft diese umgibt.

Die Figur 5 zeigt eine alternative Ausführung, bei der der letzte Knick 4 mit Bezug auf Figur 4 fehlt und der Schaft 3 direkt nach dem dritten Knick K3 in ein Kugelelement 6 übergeht, welches in der Befestigungsvorrichtung beweglich gelagert ist.

Bezüglich sämtlicher Ausführungen ist festzustellen, dass die in Verbindung mit einer Ausführung genannten technischen Merkmale nicht nur bei der spezifischen Ausführung eingesetzt werden können, sondern auch bei den jeweils anderen Ausführungen. Sämtliche offenbarten technischen Merkmale dieser Erfindungsbeschreibung sind als erfindungswesentlich einzustufen und beliebig miteinander kombinierbar oder in Alleinstellung einsetzbar.

## Patentansprüche

1. Vorrichtung zur Bestimmung der mechanischen Beinachse eines Oberschenkelknochens, mit einem Schaft (3), der an seinem einen Ende eine arretierbar winkelvariabel mit dem Schaft (3) verbundene Befestigungsvorrichtung (5) aufweist zur winkelstabil arretierbaren Befestigung des Schaftes (3) am Kniegelenk eines Oberschenkelknochens und an seinem anderen Ende ein Griffelement (10) aufweist, wobei zwischen Griffelement (10) und Schaft (3) eine Kraft-Momenten-Messvorrichtung (14) angeordnet ist, mit der Kraftkomponenten einer mittels des Griffelements (10) über den Schaft (3) auf ein Kniegelenk ausgeübten Kraft (Fz) messbar sind, **dadurch**
**gekennzeichnet, dass** mit der Kraft-Momenten-Messvorrichtung (14) Längs- und/oder Querkraftkomponenten der ausgeübten Kraft (Fz) und/oder dadurch erzeugte Momente messbar sind und die Kraft-Momenten-Messvorrichtung (14) ausgebildet ist als ein erstes ringförmiges Element (15), welches über wenigstens drei Stege (16) mit einem koaxial darin angeordneten zweiten ringförmigen Element (17) verbunden ist, wobei jeder Steg (16) wenigstens einen Kraftsensor, insbesondere Dehnungsmessstreifen aufweist.

2. Vorrichtung nach Anspruch 1, **dadurch gekennzeichnet, dass** das erste ringförmige Element (15) mit dem Griffelement (10) und das zweite ringförmige Element (17) mit dem Schaft (3) verbunden / verbindbar ist.

3. Vorrichtung nach Anspruch 1 oder 2, **dadurch gekennzeichnet, dass** das zweite ringförmige Element (17) von einem Stift (8) zur Betätigung einer Arretiervorrichtung durchdrungen ist.

4. Vorrichtung nach einem der vorherigen Ansprüche, **dadurch**
**gekennzeichnet, dass** die winkelstabil arretierbare Verbindung zwischen Schaft (3) und Befestigungsvorrichtung (5) als wenigstens ein feststellbares Gelenk, insbesondere ein feststellbares Kugelgelenk (6,7) ausgebildet ist.

5. Vorrichtung nach einem der vorherigen Ansprüche, **dadurch**
**gekennzeichnet, dass** der Schaft (3) wenigstens zweiteilig, insbesondere teleskopierbar ausgebildet ist, wobei ein Teil (3b) mit der Befestigungsvorrichtung (5) verbunden ist, ein anderer Teil (3a) mit der Griffvorrichtung (10) bzw. der Kraft-Momenten-Messvorrichtung (14) und beide Teil miteinander lösbar verbindbar sind.

6. Vorrichtung nach einem der vorherigen Ansprüche, **dadurch**
**gekennzeichnet, dass** der Schaft (3) vor der Befestigungsvorrichtung (5) geknickt (K1, K2, K3, K4) oder gebogen, insbesondere mehrfach geknickt ausgeführt ist, insbesondere so dass der Schaft (3) im Bereich der Biegung / Knickung (K1, K2, K3, K4) ein Hindernis, insbesondere eine Kniescheibe umgibt / umläuft.

7. Vorrichtung nach einem der vorherigen Ansprüche, **dadurch**
**gekennzeichnet, dass** der Schaft (3) zumindest teilbereichsweise hohl ausgebildet ist, wobei durch den hohlen Schaft (3) die Arretiervorrichtung für die Arretierung der gelenkigen Verbindung (6,7) zur Befestigungsvorrichtung (5) lösbar und/oder feststellbar ist.

8. Vorrichtung nach Anspruch 7, **dadurch gekennzeichnet, dass** im hohlen Schaft (3) ein, insbesondere mehrteiliger Stift (8) einliegt, mittels dem die Arretiervorrichtung in der Befestigungsvorrichtung (5) betätigbar ist.

9. Vorrichtung nach Anspruch 8, **dadurch gekennzeichnet, dass** der Stift (8) durch ein Betätigungselement (9) am Griffelement (10) betätigbar ist.

10. Vorrichtung nach einem der vorherigen Ansprüche, **dadurch**
**gekennzeichnet, dass** Griffelement (10) und Schaft (3), insbesondere auch das Betätigungselement (9) am Griffelement (10) pistolenartig ausgebildet sind.

11. Vorrichtung nach einem der vorherigen Ansprüche, **dadurch**
**gekennzeichnet, dass** die Kraft-Momenten-Messvorrichtung (14) lösbar zwischen Griffelement (10) und Schaft (3) angeordnet ist, insbesondere zwischen zwei Flanschen.

12. Vorrichtung nach einem der vorherigen Ansprüche, **dadurch**
**gekennzeichnet, dass** die Kraft-Momenten-Messvorrichtung (14) durch eine mechanische Vorrichtung zur Anzeige der Momente und/oder der Längskraft realisiert ist.

13. Vorrichtung nach einem der vorherigen Ansprüche , **dadurch**
**gekennzeichnet, dass** sie insgesamt oder deren Komponenten autoklavierbar ist

## Claims

1. Apparatus for determining the mechanical bone axis of a femoral bone, having a shaft (3), which at one end has a securing device (5) which is connected to the shaft (3) in a lockable manner and so as to be able to vary its angle for locking the shaft (3) at a stable angle onto the knee joint of a femoral bone and at its other end has a gripping element (10), wherein a force-torque measuring device (14) is arranged between the gripping element (10) and shaft (3) by means of which force components of a force (Fz) exerted on a knee joint by means of the gripping element (10) via the shaft (3) can be measured, **characterised in that** by means of the force-torque measuring device (14) longitudinal and/or transverse force components of the exerted force (Fz) and/or moments produced thereby can be measured and the force-torque measuring device (14) is designed as a first annular element (15) which is connected by at least three crosspieces (16) to a second annular element (17) arranged coaxially therein, wherein each cross-piece (16) has at least one force sensor, in particular strain gauges.

2. Apparatus according to claim 1, **characterised in that** the first annular element (15) is connected/connectable to the gripping element (10) and the second annular element (17) is connected/connectable to the shaft (3).

3. Apparatus according to claim 1 or 2, **characterised in that** the second annular element (17) is penetrated by a pin (8) for activating a locking device.

4. Apparatus according to any of the preceding claims, **characterised in that** the stable angle lockable connection is formed between the shaft (3) and securing device (5) as at least one lockable joint, in particular a lockable ball-and-socket joint (6, 7).

5. Apparatus according to any of the preceding claims, **characterised in that** the shaft (3) is designed in at least two parts, in particular to be telescopic, wherein one part (3b) is connected to the securing device (5), another part (3a) is connectable to the gripping device (10) or the moment of force measuring device (14) and both parts are connectable to one another in a detachable manner.

6. Apparatus according to any of the preceding claims, **characterised in that** the shaft (3) is folded or bent (K1, K2, K3, K4) before the securing device (5), in particular is bent multiple times, in particular so that in the area of the bend/fold (K1, K2, K3, K4) the shaft (3) surrounds/encloses an obstruction, in particular a kneecap.

7. Apparatus according to any of the preceding claims, **characterised in that** the shaft (3) is designed to be at least partly hollow, wherein by means of the hollow shaft (3) the locking device can be released and/or locked for locking the joint connection (6, 7) to the securing device (5).

8. Apparatus according to claim 7, **characterised in that** in the hollow shaft (3) a pin, in particular a multi-part pin (8), is inserted, by means of which the locking device can be activated in the securing device (5) .

9. Apparatus according to claim 8, **characterised in that** the pin (8) can be activated by an actuating element (9) on the gripping element (10).

10. Apparatus according to any of the preceding claims, **characterised in that** the gripping element (10) and shaft (3), in particular also the actuating element (9) on the gripping element (10), are designed to be pistol-like.

11. Apparatus according to any of the preceding claims, **characterised in that** the force-torque measuring device (14) is arranged releasably between the gripping element (10) and shaft (3), in particular between two flanges.

12. Apparatus according to any of the preceding claims, **characterised in that** the force-torque measuring device (14) is formed by a mechanical device for displaying the torques and/or the longitudinal force.

13. Apparatus according to any of the preceding claims, **characterised in that** it or its components can be autoclaved.

## Revendications

1. Dispositif de détermination de l'axe mécanique d'un fémur, avec une tige (3) qui présente, à une de ses extrémités, un dispositif de fixation (5) raccordé à la tige (3) pouvant être bloqué à des angles variables pour la fixation, pouvant être bloquée de manière stable angulairement, de la tige (3) à l'articulation de genou d'un fémur et, à son autre extrémité, un élément de préhension (10), dans lequel un dispositif de mesure de couple-force (14) est disposé entre l'élément de préhension (10) et la tige (3), avec lequel des composantes de force d'une force (Fz) exercée au moyen de l'élément de préhension (10) par le biais de la tige (3) sur une articulation de genou peuvent être mesurées, **caractérisé en ce qu'**avec le dispositif de mesure de couple-force (14) des composantes de force longitudinale et/ou transversale de la force (Fz) exercée et/ou des couples ainsi générés peuvent être mesurés et le dispositif de mesure de couple-force (14) est réalisé comme un premier élément annulaire (15) qui est raccordé à un second élément annulaire (17) disposé coaxialement dans celui-ci par le biais d'au moins trois traverses (16), dans lequel chaque traverse (16) présente au moins un capteur de force, en particulier des jauges extensométriques.

2. Dispositif selon la revendication 1, **caractérisé en ce que** le premier élément annulaire (15) est/peut être relié à l'élément de préhension (10) et le second élément annulaire (17), à la tige (3).

3. Dispositif selon la revendication 1 ou 2, **caractérisé en ce que** le second élément annulaire (17) est traversé par une broche (8) pour l'actionnement d'un dispositif de blocage.

4. Dispositif selon l'une quelconque des revendications précédentes, **caractérisé en ce que** la liaison pouvant être bloquée de manière stable angulairement entre la tige (3) et le dispositif de fixation (5) est réalisée comme au moins une articulation pouvant être bloquée, en particulier une articulation sphéroïde (6, 7) pouvant être bloquée.

5. Dispositif selon l'une quelconque des revendications précédentes, **caractérisé en ce que** la tige (3) est réalisée au moins en deux parties, en particulier de manière télescopique, dans lequel une partie (3b) est reliée au dispositif de fixation (5), une autre partie (3a) peut être reliée au dispositif de préhension (10) ou au dispositif de mesure de couple-force (14) et les deux parties peuvent être reliées l'une à l'autre de manière détachable.

6. Dispositif selon l'une quelconque des revendications précédentes, **caractérisé en ce que** la tige (3) est réalisée de manière pliée (K1, K2, K3, K4) ou courbée, en particulier de manière pliée à plusieurs reprises, avant le dispositif de fixation (5), en particulier de sorte que, dans la zone du pliage/de la courbe (K1, K2, K3, K4), la tige (3) entoure/enferme un obstacle, en particulier une rotule.

7. Dispositif selon l'une quelconque des revendications précédentes, **caractérisé en ce que** la tige (3) est réalisée au moins partiellement par endroits de manière creuse, dans lequel le dispositif de blocage peut être détaché et/ou bloqué par la tige creuse (3) pour le blocage de la liaison articulée (6, 7) avec le dispositif de fixation (5).

8. Dispositif selon la revendication 7, **caractérisé en ce qu'**une tige (8) en particulier en plusieurs parties est logée dans la tige creuse (3), au moyen de laquelle le dispositif de blocage peut être actionné dans le dispositif de fixation (5).

9. Dispositif selon la revendication 8, **caractérisé en ce que** la tige (8) peut être actionnée par un élément d'actionnement (9) au niveau de l'élément de préhension (10).

10. Dispositif selon l'une quelconque des revendications précédentes, **caractérisé en ce que** l'élément de préhension (10) et la tige (3), en particulier aussi l'élément d'actionnement (9) sont réalisés comme un pistolet.

11. Dispositif selon l'une quelconque des revendications précédentes, **caractérisé en ce que** le dispositif de mesure de couple-force (14) est disposé de manière détachable entre l'élément de préhension (10) et la tige (3), en particulier entre deux brides.

12. Dispositif selon l'une quelconque des revendications précédentes, **caractérisé en ce que** le dispositif de mesure de couple-force (14) est réalisé par un dispositif mécanique pour l'affichage des couples et/ou de la force longitudinale.

13. Dispositif selon l'une quelconque des revendications précédentes, **caractérisé en ce qu'**il peut être autoclavé dans l'ensemble ou ses composants.
